# EUROPEAN PATENT APPLICATION

(11) **EP 1 201 139 A1**
(43) Date of publication of application: **02.05.2002**
(21) Application number: 00830705.0
(22) Date of filing: 26.10.2000
(51) Int. Cl.: A23L 3/015, A61L 2/00, A23L 2/02, F01B 31/00, B01J 3/04

(54) **Pressurization Device**

(71) Applicant: SIG SIMONAZZI BEVERAGE S.P.A., 43040 Parma (IT)
(72) Inventor: Zacche, Vanni, 43040 Parma (IT); Dalcielo, Massimiliano, 43040 Parma (IT); Preti, Fabrizio, 43040 Parma (IT)
(74) Representative: Long, Giorgio

(57) **Abstract**

The present invention relates to a device for the high-pressure sterilization of packaged foodstuffs, in particular bottled drinks, a high-pressure sterilization process and an apparatus for carrying out this process.

More specifically, the present invention relates to a pressurization device (1) comprising a jacket (4) with a closure (3) and containing a pressurization chamber (18), the said pressurization chamber (18) being connected to pressurization means (47, 48), the said pressurization device (1) being characterized in that the said closure (3) is located at the top of the device for the top loading of an object that is to be pressurized.

## Description

The present invention relates to a pressurization device, a process for sterilizing packaged foodstuffs at high pressure and an apparatus for carrying out this process.

Many different methods of sterilization/pasteurization of foodstuffs and their containers exist in the industry.

One method is to sterilize the foodstuffs or drinks by heat treatment and package them at high temperature in order to make use of their heat energy to sterilize the container. This method can be used in containers capable of not changing their physical or chemical characteristics at the filling temperature and it is usually used with containers made of glass or aluminium (cans) or recrystallized PET.

Another method is to sterilize the foodstuffs by heat treatment prior to packaging, which is then carried out at room temperature, in an aseptic environment, in a presterilized container. This system presupposes that the container is sterilized with chemical/physical agents and that an aseptic environment is maintained during packaging and sealing by employing special sterile chambers (known as "clean rooms") with a volume of several cubic metres. Such rooms are difficult to run in industrial environments.

The growing popularity in recent years of new dietary products, with added vitamins and/or important trace elements to add to the diet has created a need for sterilization methods other than heat in order to avoid irreversible processes of denaturation of the product.

Of the various methods employed - use of pulsed electric fields, magnetic fields, microwaves, etc - particular interest has recently been aroused by the method based on the use of hyperbaric chambers in which sterilization is carried out by treatment at a high hydrostatic pressure. It is known that the application of the high pressure causes morphological changes in the cells of the microorganisms, even to the extent of rupturing the cell membrane and so killing the microorganism.

The magnitude and speed of the changes brought about by the high pressure depends on a number of factors, including the magnitude of the pressure itself, the application time and the type of pressure/decompression cycle used.

The application of the method of sterilization by hyperbaric pressure to the food industry is known. It has however the disadvantage of being discontinuous as it requires the use of large hyperbaric chambers into which a batch of products are inserted for sterilization. In addition, it is characterized by the long sterilization cycle times which are due in part to the time required for the apparatus to reach the operating pressure and return at the end to atmospheric pressure. It is obvious therefore that the known method is of limited versatility and does not allow the equipment to be sufficiently productive.

To overcome these problems an apparatus has been proposed for the high-pressure sterilization of drinks in which a continuous linear or rotary apparatus comprises a plurality of high-pressure sterilization chambers, each designed to take one bottle. This solution is covered by European patent application No. 99 830 254.1 filed on 29 April 1999.

Even this apparatus, while largely solving the problems inherent in the apparatus and processes of the prior art, nevertheless has a number of drawbacks. Specifically, the sterilization device as disclosed in the abovementioned patent application is of complicated operation, particularly as regards the mechanism for placing the bottle in the device and removing it.

Furthermore, the releasing of the bottle in the final stage of the process occurs by gravity and causes the fluid contained in the pressurization chamber to escape, which fluid is then collected in a gutter located underneath the apparatus. This approach has a number of disadvantages. In the first place, it would be preferable for the bottle not to be dropped under gravity but held in a positive grip, for example by suitable pincers or grabs, so as to facilitate its transfer to other processing stages. Furthermore, the escape of all the fluid contained in the sterilization chamber requires a recirculating pump of adequate size, which is an additional burden to the operating costs. Another disadvantage of the loss of all the fluid from the pressurization chamber after each working cycle is that the time spent returning it detracts from the optimization of the entire process.

The problem addressed by the present invention is therefore to provide a pressurization device that overcomes the above disadvantages.

This problem has been solved by a pressurization device as delineated in the attached claims.

Other characteristics and advantages of the pressurization device that forms the subject-matter of the present invention will become apparent in the course of the following description of certain preferred embodiments. The description is given by way of non-restrictive guidance and refers to the following figures, in which:
Figure 1 is a perspective view of the pressurization device to which the present invention relates, in the open condition;
Figure 2 is a side view in section of the device shown in Figure 1;

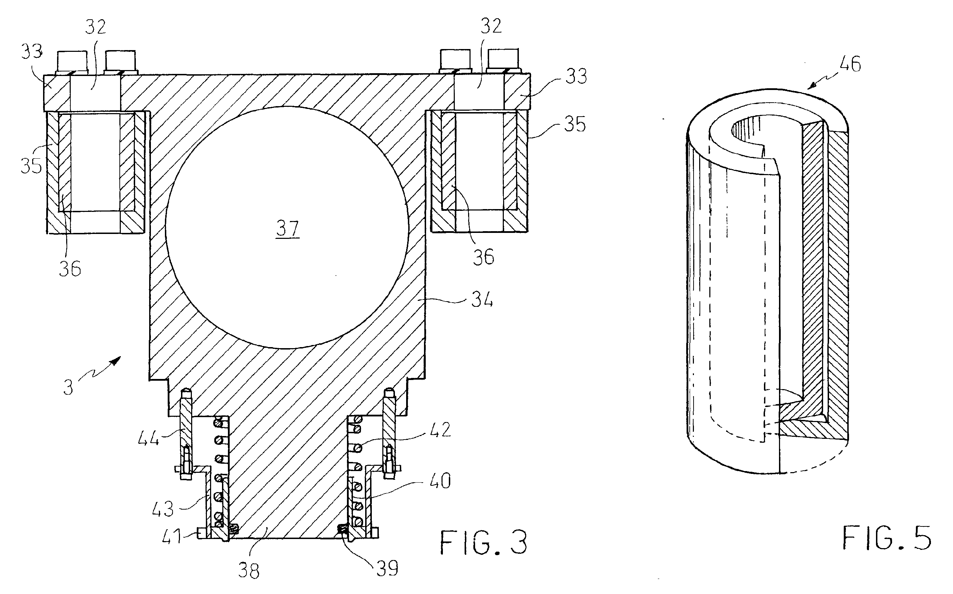

Figure 3 is a side view in section on III-III as marked in Figure 2 of a detail of the pressurization device to which the present invention relates;
Figure 4 is a side view in section of a second embodiment of the pressurization device of the invention, in the closed condition; and
Figure 5 is a perspective view in partial section of a series of volume reducers according to the present invention.

Referring to the figures, the pressurization device according to the present invention, which has the general number 1, basically comprises a hollow casing 2 and a lid 3 connected movably to the said casing 2.

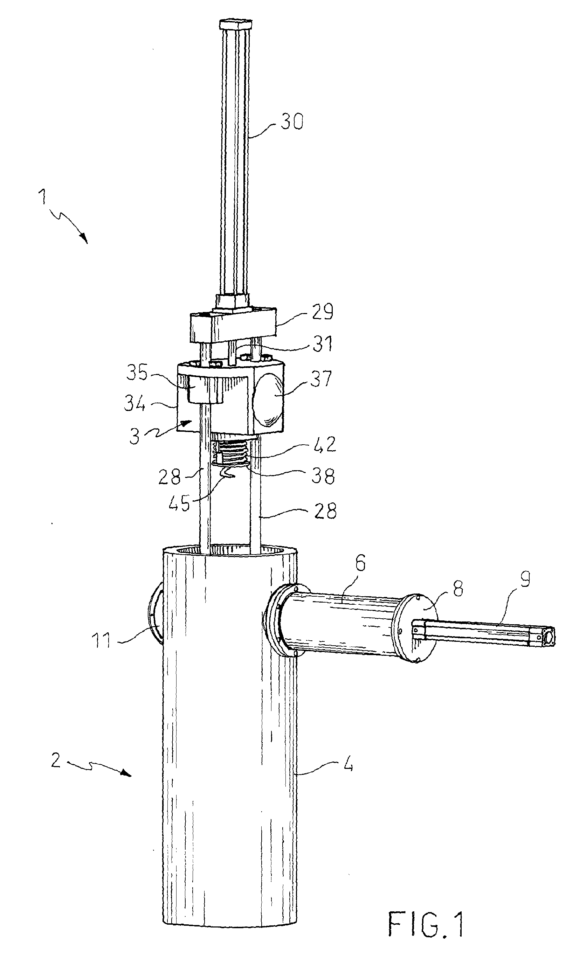

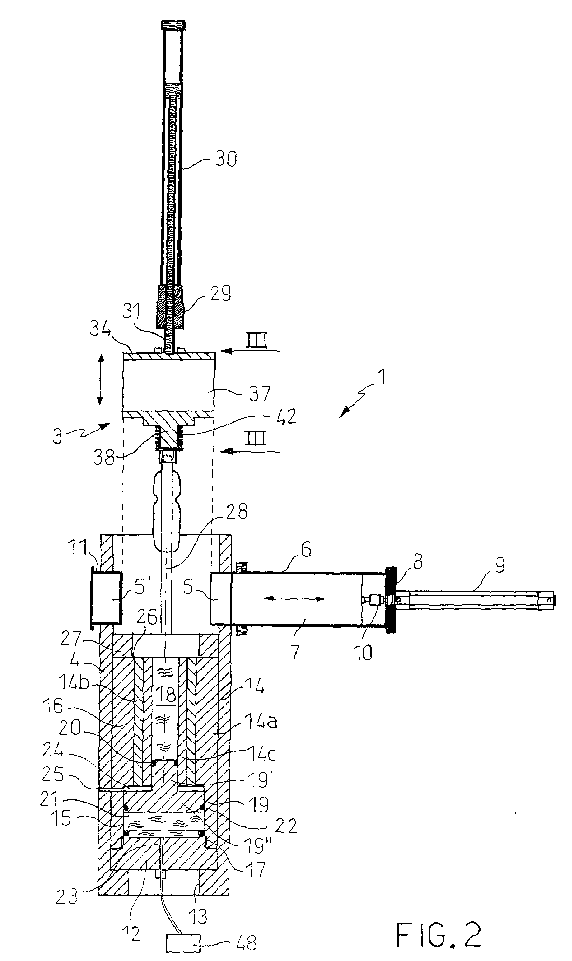

As shown in Figures 1 and 2, the hollow casing 2 comprises a basically cylindrical jacket 4 open at both ends. Two holes 5, 5' are prepared in mutual alignment in the cylindrical lateral surface of the jacket 4, in the portion near the upper end of the jacket where the lid 3 is to go.

Fastened to the first hole 5 is a cylindrical guide 6 for a cylinder 7 for locking the lid 3, as will be explained later in the description. The end of the guide 6 inside the jacket 4 is open, while the other or outer end is closed by a closing disc 8. Mounted on the outside of the disc 8 is an actuator 9, preferably a linear actuator, and more preferably a brushless motor or a hydraulic cylinder. The disc 8 has a central hole for the passage of the actuator 9 shaft 10, which in turn is fastened to the outer end of the cylinder 7.

Meanwhile a sleeve 11 is fastened to the second hole 5' with its outer end closed and its inner end open.

The inside diameter of the guide 6 and of the sleeve 11 is approximately equal to and corresponds to the diameter of the cylinder 7, thereby allowing the latter to slide inside them. The guide 6 and the sleeve 11 moreover lie on the same axis, which is essentially perpendicular to the main axis of the jacket 4.

The jacket 4 is sealed at the bottom by a cylindrical T-section closing element 12 whose major base stands on the annular shoulder 13 formed in and projecting inwards from said bottom end of the jacket 4.

Fitted inside the jacket 4, immediately above the closing element 12, is a multilayer liner 14, comprising an outer layer 14a, an intermediate layer 14b and an inner layer 14c. The three layers are force-fitted inside each other by a process of fitting two adjacent layers in which the outer layer is heated so that it expands, and the inner layer is cooled so that it contracts. When the temperature of the two layers has returned to equilibrium at room temperature, the two adjacent layers will exert pressure on each other such that they behave as if they were radially prestressed. The operation is repeated between the two fitted layers and the third layer, thus producing the multilayer liner 14. The multilayer structure described above has a mechanical resistance to the stresses induced by the high pressure created inside the device that is greatly superior to the mechanical strength of a single-layer liner of the same thickness. It is thus possible to produce a pressurization chamber capable of withstanding many thousands of atmospheres within a structure of limited weight and size. Both the jacket 4 and the multilayer liner 14 are of course also made from suitable materials capable of withstanding the high pressures.

The outer layer 14a of the multilayer liner 14 has a lower portion 15 and an upper portion 16, the lower portion 15 being thinner than the upper portion 16. The end of the said lower portion 15 fits between the inner surface of the jacket 4 and the closing element 12 at the meeting point between the two portions of different diameter of the latter. On the inner surface of the said end of the lower portion 15 of the outer layer 14a an annular seal 17 interacts with the cylindrical surface of the smaller-diameter section of the closing element 12 to prevent leaks.

The intermediate layer 14b is force-fitted onto the inner surface of the upper portion 16 of the outer layer 14a by the process described above, and the inner layer 14c is force-fitted inside that. The intermediate 14b and inner 14c layers therefore have less height than the outer layer 14a, corresponding essentially with only the upper portion 16 of the latter.

Defined inside the said multilayer liner 14 is a cylindrical pressurization chamber 18 open at both ends, its lower opening being closed by a piston 19 in the form of an inverted T. The smaller-diameter portion 19' of the piston 19 slides against the inner surface of the multilayer liner 14, which forms the cylindrical wall of the pressurization chamber 18. An annular seal 20 placed around the surface of the said portion 19' prevents leaks out of the pressurization chamber 18.

The larger-diameter portion 19" of the said piston 19 is housed in the space formed above the closing element 12 and underneath the shoulder 24 situated between the lower portion 15 and the upper portion 16 of the outer layer 14a of the multilayer liner 14. Because the volume of the portion 19" of the piston 19 is smaller than the volume of the said space, a reservoir 21 containing a substantially incompressible fluid, such as hydraulic oil, is defined between the piston 19 and the closing element 12.

Between the annular surface of the piston 19 and the shoulder 24 there exists, however, a gap that defines the piston stroke. This gap is in communication with the external environment via an air bleed 25.

The larger-diameter portion 19" of the piston 19 accommodates, around its cylindrical surface, an annular seal 22 which, together with the seal 17 described earlier, prevents leaks from the reservoir 21.

Owing to its T shape, whereby the smaller-diameter end surface of the said piston is towards the interior of the pressurization chamber 18, the piston 19 acts as a pressure multiplier, the pressure reached inside the pressurization chamber 18 being proportional to the ratio between the upper and lower surfaces of the piston.

The hydraulic oil reservoir 21 is connected to pressurization means. In the example of Figure 2, a line 23 passes through the closing element 12 and is connected externally to a hydraulic power unit (not shown in the figure).

On the upper annular surface 26 of the multilayer liner 14 is a ring 27 that ends below the guide 6 and sleeve 11. At the ends of a diameter across this ring 27 are fixed two rods 28 that extend upwards outside of the jacket 4. The outer ends of these rods 28 are fastened to a plate 29, on whose upper surface is mounted an actuator 30, preferably a linear actuator. The shaft 31 of the actuator 30 runs through a hole in the plate 29 and so protrudes below the plate 29.

The shaft 31 of the actuator 30 is attached to the upper surface of the lid 3. This upper surface of the lid has two more through holes 32, in the fins 33 projecting from the body 34 of the lid 3. Two bushes 35, each acting as a support to a linear ball bearing 36 on its inside, are fastened (by a screw and nut system in the example) underneath and in line with these holes 32. This system comprising the holes 32, the bushes 35 and the associated linear ball bearings 36 serves to contain the rods 28, which can slide through them and therefore act as guides for the lid 3 during the vertical movement of closing/opening the jacket 4.

The body 34 of the lid 3 contains a through hole 37 whose diameter essentially corresponds to the diameter of the cylinder 7 and it is arranged, when the lid is closed, in line with the cylinder. Furthermore, the axial length of the hole 37 essentially corresponds to the distance between the inward ends of the guide 6 and sleeve 11.

Below the body 34 of the lid 3 extends an essentially cylindrical plug element 38 whose diameter essentially corresponds to that of the pressurization chamber 18. An annular seal 39 is housed in a seat formed for it around the surface of the plug element 38, in the vicinity of the lower end of the latter. A cylinder 40 is fitted around the said annular seal 39, to keep it pressed against the surface of the plug element 38. At the lower end of the cylinder 40 is a flange 41 with a spring 42 acting on it, this spring being placed between the flange 41 and the lower surface of the body 34 of the lid 3.

The flange 41 of the cylinder 40 is fixed to two L brackets 43, in the upper end of each of which is a through hole for the pin 44 to slide through. The pin 44 has a washer 44' on its lower end for axial retention of the bracket.

Attached to the underside of the plug element 38 are means of engagement 45 with the container. The said means of engagement 45 are of known type and, in the example of the figure, take the form of a hook.

The pressurization device that forms the subject-matter of the present invention also includes a pressure sensor and, optionally, a temperature sensor placed inside the pressurization chamber 18, these sensors being connected to a command and control unit that constantly monitors the operational conditions of the system. It is therefore possible to monitor the sterilization process control to ensure that it is running correctly and consequently remove any defective container or, in the event of a repeated processing error, identify the fault in a device.

The pressurization device may also be equipped with energy recovery means (not shown in the figures), the job of which is to recover some of the energy released by the system during the decompression phase that takes place at the end of the pressurization cycle. In the example shown in Figure 2, in which pressurization is effected by means of a hydraulic power unit comprising a pump that injects the hydraulic oil at high pressure into the reservoir 21, energy can be recovered by a turbine connected to a dynamo or by means of gas-type potential energy accumulators.

Another characteristic of the pressurization device that forms the subject-matter of the present invention is that it includes, as shown in Figure 5, one or more volume reducers 46 for insertion into the pressurization chamber 18, the job of these being to adapt the volume of the pressurization chamber to the actual dimension of the container being processed, which may be different in different cases. It is thus possible to reduce to a minimum the amount of fluid outside the container that has to be injected into the pressurization chamber, and thereby optimize the pressurization process.

The volume reducer 46 is in the form of a cylinder closed at its base and open at its top, with an outside diameter approximately equal to or slightly less than the diameter of the pressurization chamber 18 and an inside diameter slightly smaller than the maximum diameter of the container.

As shown in Figure 5, a second volume reducer 46 can be inserted in the first to adapt the volume of the pressurization chamber to even smaller containers. Alternatively, another possibility would be to provide volume reducers specifically adapted to the different types of container, i.e. all having the same outside diameter but with inside diameters tailored to the size of the container. In other cases the reducer 46 will be open at both ends.

The volume reducer 46 may be made of materials of different types, such as metals, metal alloys or plastic materials.

The pressurization device 1 according to the present invention may be part of a foodstuff packaging apparatus, e.g. to carry out the sterilization stage, but also for other types of process requiring treatment at high pressure. Such apparatus may be either linear or rotary and will incorporate one or more movable pressurization devices according to the invention. An example of an apparatus to which the pressurization devices according to the present invention can be applied is disclosed in European patent application No. 99 830 254. 1 filed on 29 April 1999. The apparatus will include means for continually filling the inside of the pressurization chamber with water (or other substantially incompressible fluid), in such a way as to keep it full to the brim at all times throughout the process.

Referring again to the figures, the manner in which the pressurization device forming the subject-matter of the present invention works will now be described. The action of transferring the container, a bottle in the drawing, from the conveying system of the apparatus to the device 1 has not been described as it is perfectly conventional. The device is therefore shown in Figure 2 with the bottle already held in the engagement means 45 of the lid 3, immediately prior to the pressurization stage.

At this point the actuator 30 will act on the lid 3 to cause it to descend along the rods 28 which, as stated, act as guides. The lid 3 will be lowered until the hole 37 is aligned with the cylinder 7. The actuator 9 will then cause the latter to advance through the said hole 37 and into the sleeve 11. In this way the cylinder 7 acts as a lock on the lid 3 to contain the axial pressure generated during pressurization.

During the descent of the lid 3 and before the hole 37 of the lid is aligned with the cylinder 7, the bottle enters the pressurization chamber until the flange 41 of the ring 40 interacts with the upper edge of the pressurization chamber and brings it to a stop. The lid continues to descend - thereby putting the spring 42 under load-thus inserting the annular seal 39 end of the plug element 38 into the pressurization chamber 18. The seal 39, which until its entry into the pressurization chamber was compressed by the cylinder 40, will pass into the said pressurization chamber without coming under mechanical stress and will maintain the same compression to which it was subject previously, thereby exerting a sealing pressure against the walls of the pressurization chamber. It is only at this point that the hole 37 of the lid 3 and the cylinder 7 are aligned.

The bottle is now therefore sealed inside the pressurization chamber 18 and completely immersed in water. At this point the pump of the hydraulic power unit will feed the oil into the reservoir 21 of the device at a predetermined pressure. According to Pascal's law, this pressure will be transmitted to the surface of the piston 19, which will therefore advance until equilibrium is established with the force of reaction exerted by the fluid present in the pressurization chamber 18. As stated earlier, the piston 19 of inverted T section serves as a pressure multiplier. This means that the pressure exerted inside the pressurization chamber is, at least to a first approximation, multiplied, as compared with the pressure initially imparted by the hydraulic power unit, by an amount equal to the ratio between the lower and upper end surfaces of the said piston 19. Consequently, given a certain starting pressure imparted by the hydraulic power unit, it is possible to obtain hydrostatic pressures of up to 10 000 bar inside the pressurization chamber 18, simply by modifying the ratio between the areas of the two faces of the piston 19.

Decompression is brought about simply by interrupting the action of the hydraulic power unit. This causes a rapid return of the pressure to equilibrium with atmospheric pressure. The piston 19 recedes and the oil initially pumped into the reservoir 21 retreats, actuating the energy recovery means described earlier. The operation can be repeated, carrying out a cycle of compressions and decompressions, preferably lasting not more than 60 seconds, which kills the microorganisms present in the foodstuff or drink in an extremely short time.

At the conclusion of the pressurization stage, and with the pressure in the pressurization chamber 18 back at atmospheric pressure, the actuator 9 withdraws the cylinder 7, which frees the lid 3. The latter is then raised by the actuator 30, thus allowing the bottle to be extracted and a new cycle to be commenced.

The command and control unit, as stated earlier, operates the actuators and the hydraulic power unit in accordance with the predefined program and monitors the entire process to pick up any malfunctions and faults.

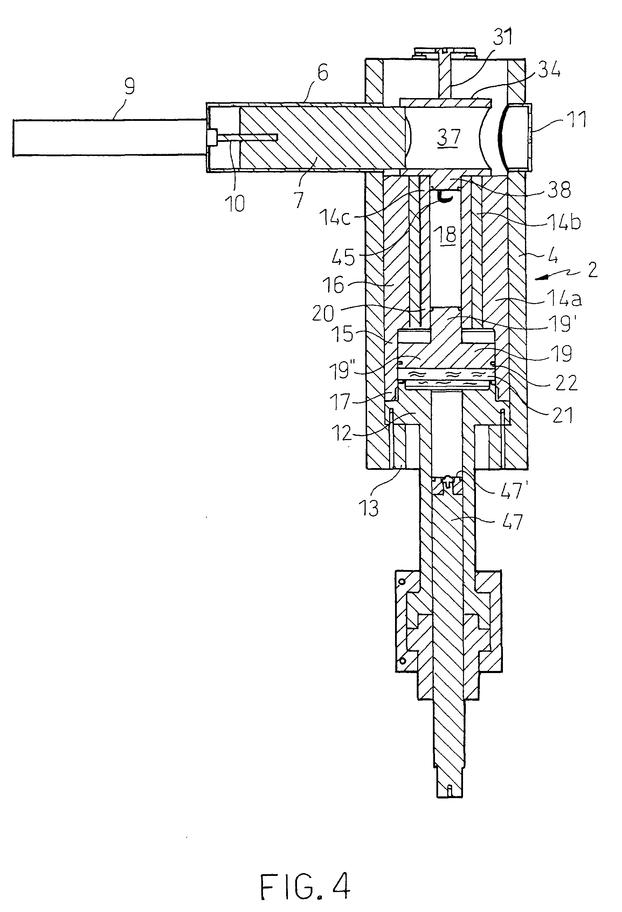

Figure 4 shows a second embodiment of the present invention. In the figure, the lid is already inserted in the jacket 4 while the cylinder 7 is in the process of advancing. The upper portion of the device, comprising the rods 28, the plate 29 and the actuator 30, has been omitted from the drawing, but it must of course be considered an integral part of the present embodiment of the invention, as shown in Figures 1 and 2 outlined above.

The embodiment shown in Figure 4 is therefore exactly analogous to the first embodiment described earlier, with the sole difference that the pressurization means consist of a screw system 47 which acts directly on the reservoir 21 and so transmits the pressure to the piston 19. The upper end 47' of the screw 47 has a seal to ensure leaktightness with respect to the reservoir 21.

The advantages of the pressurization device forming the subject-matter of the present invention are obvious. In the first place, its structure is lightweight and small when considered in relation to the extreme operating conditions to which it is designed to be subjected. The jacket 4 serves to contain the axial loads, while the multilayer liner 14, which has high resistance to radial loads, is limited to the walls of the pressurization chamber 18. The multilayer liner 14 also has a strength which, for a given weight, is greatly superior to that of a solid steel liner.

The closing/opening system of the device is cinematically simple and therefore unlikely to give trouble. It is also suitable for fast working.

The loading of the container from the top down reduces the amount of water required to refill the pressurization chamber, all that is required being the very small amount of water necessary to make up for the water that inevitably overflows during each operating cycle. In this way time is saved and the recirculating pump can be smaller.

The command and control unit connected to the sensors of pressure and optionally of temperature (an internal temperature rise is inevitable during the compression phase) ensure that the system runs well by detecting faults and malfunctions.

The energy recovery means associated with the pressurization means help to optimize the process from the standpoint of energy efficiency. Similarly, the provision of the volume reducers 46 enables the volume of fluid to be pressurized inside the pressurization chamber 18 to be reduced.

The sterilization process that can be performed with the pressurization device according to the present invention is such that it can also be used on foodstuffs or drinks that would not withstand known processes without deterioration. A typical example is its application to drinks such as milk or beer.

Clearly, only two particular embodiments of the pressurization device that is the subject-matter of the present invention have been described, and those skilled in the art will be able to make any modifications necessary for its adaptation to particular applications, without thereby departing from the scope of the protection of the present invention.

For instance, the shape of the pressurization chamber could be adapted to the type of container which it is wished to process, which may not necessarily be a bottle but for example a pot or the like.

The pressurization means and the pressure multiplying means could be tailored to give the desired predefined pressure to the fluid in the pressurization chamber. The structure of the device could be modified for greater resistance to the pressures generated.

The invention described above has a series of characteristics that could also be adapted to the pressurization devices of the prior art. For example, the use of the jacket 4 to contain axial loads, and of the multilayer liner 14 to contain radial loads, are characteristics that can be adapted to all those devices designed for processes in which a period of high pressure is required.

The same can be said as regards the energy recovery means and the volume reducers 46.

The system of precompression of the seal 39 by the cylinder 40 makes it possible, as indicated, to reduce the mechanical stress on this seal when inserted into the pressurization chamber 18. The system can therefore advantageously be applied to all those situations in which a leaktight closure must be inserted in a container or other receptacle.

## Claims

1. Pressurization device (1) comprising a jacket (4) with a closure (3) and containing a pressurization chamber (18), the said pressurization chamber (18) being connected to pressurization means (47, 48), the said pressurization device (1) being **characterized in that** the said closure (3) is located at the top of the device for the top loading of an object that is to be pressurized.

2. Device according to Claim 1, in which the said device comprises locking means (7) for locking the said closure (3).

3. Device according to Claim 1 or 2, in which the said pressurization chamber (18) is radially contained in a multilayer liner (14).

4. Device according to Claim 3, in which the said multilayer liner (14) comprises an inner layer (14c), an intermediate layer (14b) and an outer layer (14a), the said outer layer (14a) being in engagement with the inner surface of the said jacket (4).

5. Device according to Claim 4, in which the said inner (14c), intermediate (14b) and outer (14a) layers of the said multilayer liner (14) are force-fitted inside each other.

6. Device according to any one of Claims 1 to 5, in which the said pressure multiplier means are a T-section piston (19), the smaller-diameter end surface of the said piston being directed into the said pressurization chamber (18).

7. Device according to any one of Claims 1 to 6, in which the said pressurization means consist of a hydraulic power unit (48) equipped with a pump, the said hydraulic power unit being connected to the hydraulic oil reservoir (21) of the pressurization device (1) by a line (23), the said pressure multiplier means (19) being between the said reservoir (21) and the said pressurization chamber (18).

8. Device according to any one of Claims 1 to 6, in which the said pressurization means consist of a screw (47) acting directly on the said reservoir (21) of the said device (1).

9. Device according to any one of Claims 1 to 8, in which the said closure is a lid (3) comprising a body (34) having a through hole (37) and the said lid (3) locking means comprise a cylinder (7) that slides inside a guide (6) and, in alignment with the said cylinder (7) and at a diametrically opposite position on the jacket (4), a sleeve (11), the said through hole (47) and the said cylinder (7) being in alignment when the lid is closed.

10. Device according to any one of Claims 1 to 9, in which the said closure (3) and the said closure locking means (7) are operated independently by respective actuators (30, 9).

11. Device according to any one of the preceding claims, in which the said lid (3) comprises a plug element (38), with engagement means (45) for the container that is to be processed.

12. Device according to any one of the previous claims, in which the said plug element (38) comprises an annular seal (39), a cylinder (40) being fitted around the said annular seal (39) to keep it pressed flush with the surface of the plug element (38).

13. Device according to Claim 12, in which the said cylinder (40) has a flange (41) acted upon by a spring (42), this spring being placed between the flange (41) and the underside of the body (34) of the closure (3).

14. Device according to Claim 13, in which the flange (41) of the cylinder (40) is attached to two L brackets (43), at the upper end of each of which is a through hole for the pin 44 to slide through, the said pin 44 having a washer (44') at its lower end for the axial retention of the brackets.

15. Device according to any one of Claims 1 to 14, the said device comprising energy recovery means connected to the said pressurization means (47, 48).

16. Device according to Claims 7 and 15, in which the said energy recovery means comprise a turbine connected to a dynamo or gas-type potential energy accumulator.

17. Device according to Claims 8 and 15, in which the said energy recovery means comprise a device that converts mechanical energy into electrical energy.

18. Device according to any one of Claims 1 to 17, the said device comprising pressure sensors and, optionally, temperature sensors connected to a command and control unit.

19. Device according to Claim 18, in which the said command and control unit operates the actuators (30, 9) and the pressurization means according to a predefined program and monitors the operating conditions of pressure and, optionally, temperature of the process.

20. Device according to any one of Claims 1 to 19, in which the said pressurization device (1) comprises one or more volume reducers (46) of form and dimensions such that they can be inserted in the said pressurization chamber (18).

21. Apparatus for carrying out a high-pressure treatment on objects to be processed, **characterized in that** it comprises one or more pressurization devices (1) according to any one of Claims 1 to 20.

22. Apparatus according to Claim 21, the said apparatus comprising fluid refilling means for the said pressurization chamber (18).

23. Process of sterilization at high hydrostatic pressure, comprising the following steps:
- making an apparatus as described in Claim 21 or 22 available;
- placing a container in each pressurization device (1);
- carrying out a cycle of compressions and decompressions in the said pressurization chamber (18) of the said pressurization device (1);
- and removing the containers from each of the said pressurization devices (1).

24. Sterile drink or foodstuff obtainable by means of the sterilization process according to Claim 23.

25. Drink according to Claim 24, in which this drink is beer or milk.

26. Pressurization device (1) comprising a pressurization chamber (18), **characterized in that** the said pressurization chamber (18) has preferably cylindrical side walls made from a multilayer liner (14), the said multilayer liner (14) comprising an inner layer (14c), an intermediate layer (14b) and an outer layer (14a), the said layers being force-fitted inside each other.

27. Pressurization device (1) comprising a pressurization chamber (18) having walls capable of withstanding high pressures and a closure (3) equipped with locking means (7), **characterized in that** the said pressurization device (1) is externally covered by a jacket (4) on which the said locking means (7) are located to contain the axial loads.

28. Pressurization device (1) comprising a pressurization chamber (18) for treatment at high hydrostatic pressure, **characterized in that** it includes one or more volume reducers (46) whose form and dimensions are such that they can be inserted in the said pressurization chamber (18).

29. Pressurization device (1) comprising a pressurization chamber (18) and pressurization means (47, 48), the said device comprising energy recovery means connected to the said pressurization means (47, 48).

30. Container comprising a closure (3) having a plug element (38) fitted with an annular seal (39), **characterized in that** a sliding cylinder (40) is fitted around the said annular seal (39) to keep it pressed flush with the surface of the plug element (38).
